Europäisches Patentamt

European Patent Office

Office européen des brevets

(11) Publication number: **0 458 070 A1**

# EUROPEAN PATENT APPLICATION

(21) Application number: 91106397.2

(22) Date of filing: **20.04.91**

(51) Int. Cl.5: **C12N 15/52**, C12N 9/00,
C12P 19/38, //(C12N15/52,
C12R1:07)

The applicant has filed a statement in accordance with Rule 28 (4) EPC (issue of a sample only to an expert). Accession number(s) of the deposit(s): FERM BP-2860, FERM BP-2859 and FERM BP-2861.

(30) Priority: **23.04.90 JP 106721/90**

(43) Date of publication of application:
**27.11.91 Bulletin 91/48**

(84) Designated Contracting States:
**DE ES FR IT**

(71) Applicant: **TAKEDA CHEMICAL INDUSTRIES, LTD.**
**3-6, Doshomachi 2-chome Chuo-ku**
**Osaka 541(JP)**

(72) Inventor: **Asahi, Satoru**
**1-317, 5 Dooucho 5-chome**
**Takatsuki, Osaka 569(JP)**
Inventor: **Doi, Muneharu**
**11-11 Hirai 2-chome**
**Takarazuka, Hyogo 665(JP)**

(74) Representative: **von Kreisler, Alek,**
**Dipl.-Chem. et al**
**Patentanwälte Von Kreisler-Selting-Werner,**
**Deichmannhaus am Hauptbahnhof**
**W-5000 Köln 1(DE)**

(54) Cytidine analogue resistance gene DNA and its use.

(57) DNA containing cytidine 5'-triphosphate synthetase gene obtained from chromosomal DNA of the genus Bacillus exhibiting cytidine analogue resistance. A plasmid in which the DNA is incorporated, a bacterial strain transformed with the plasmid, particularly, that belonging to the genus Bacillus having capability of the production of cytidine, and a process for production of cytidine by cultivating the bacterial strain are also disclosed.

EP 0 458 070 A1

## FIELD OF THE INVENTION

The present invention relates to a process for production of cytidine which is a substance of importance as a biochemical agent or a raw material for synthesis of medicaments.

## BACKGROUND OF THE INVENTION

In order to produce cytidine by fermentation, a method wherein artificial mutation is caused for a wild type strain to impart a capacity to produce cytidine is employed because the wild type strain hardly produces cytidine. Heretofore, various processes for fermentative production of cytidine have been known, for example, there have been known a process using a mutant of Bacillus subtilis or Proteus rettgeri - (Japanese Patent Laid-Open Publication No. 21499/1961); a process using a purine analogue resistant strain, a pyrimidine analogue resistant strain and a histidine analogue resistant strain derived from bacteria belonging to the genus Brevibacterium (Japanese Patent Publication Laid-Open No. 18871/1982); a process using a purine analogue resistant strain derived from bacteria belonging to the genus Microbacterium - (Japanese Patent Laid-Open Publication No. 18872/1982); a process using a strain deficient in cytidine deaminase activity and having pyrimidine analogue resistance which is derived from bacteria belonging to the genus Bacillus (Japanese Patent Laid-Open Publication No. 135597/1986).

## OBJECTS OF THE INVENTION

As for improvement of cytidine producers, the above techniques have been known. However, the conventional mutation treatment has no sound theoretical basis for increasing the yield by selective amplification of the specific gene.

## BRIEF EXPLANATION OF DRAWINGS

Fig. 1 illustrates a restriction enzyme map of plasmid pYRG100 obtained in Example hereinafter.

Fig. 2 illustrates procedure and a restriction enzyme map of plasmid pINV100 obtained in Example hereinafter.

Fig. 3 illustrates a restriction enzyme map of plasmid pINV128 obtained in Example hereinafter.

In the drawings, the symbols P, E, PII, M and C represent cleavage sites by restriction enzymes, PstI, EcoRI, PvuII, MspI and ClaI, respectively. The symbols Cm$^r$, Am$^r$ and Tc$^r$ show regions of chloramphenicol resistance gene, ampicillin resistance gene and tetracycline resistance gene, respectively. The blank part is vector DNA and the black part means chromosomal DNA fragment derived from Bacillus subtilis DFCR-100.

## SUMMARY OF THE INVENTION

The present inventors have studied intensively to develop cytidine producers by using gene recombination technique. As a result, the present inventors have succeeded in isolating a gene region coding for cytidine 5'-triphosphate synthetase gene (hereinafter referred to as CTP synthetase gene) from bacteria belonging to the genus Bacillus showing cytidine analogue resistance, and have been found that the region provides cytidine analogue resistance and a transformant obtained by using a plasmid in which the gene region is incorporated can accumulate a large amount of cytidine in a medium. Thus, the present invention has been completed.

That is, the present invention provides (1) DNA containing CTP synthetase gene obtained from chromosomal DNA of a bacterial strain of the genus Bacillus exhibiting cytidine analogue resistance, especially, DNA containing CTP synthetase gene providing cytidine analogue resistance; (2) plasmid in which the DNA is incorporated; (3) a bacterial strain transformed with the plasmid, especially, a bacterial strain of the genus Bacillus having capability of the production of cytidine; and (4) a process for production of cytidine which comprises cultivating a bacterial strain of the genus Bacillus transformed with plasmid in which CTP synthetase gene providing cytidine analogue resistance is incorporated to produce and accumulate cytidine in a culture medium, and collecting the resultant.

## DETAILED DESCRIPTION OF THE INVENTION

The DNA used in the present invention exhibits cytidine analogue resistance and can be isolated from bacteria of the genus Bacillus having capability of the production of cytidine. Such a DNA is also produced by inserting CTP synthetase gene from wild-type bacteria belonging to Bacillus into a plasmid vector and mutation of the resulting recombinant DNA. Example of the DNA donor includes Bacillus subtilis DFCR-100 (IFO 15026, FERM BP-2860) which is a cytidine analogue resistant mutant derived from Bacillus subtilis No. 122 (IFO 14386, FERM P-7908). The cytidine analogue resistant strain used herein means a mutant derived from a bacterial strain belonging to the genus Bacillus as a parent, which can grow on a medium containing cytidine analogue in such a concentration that the parent strain can not grow. A cytidine analogue used herein means a material whose growth inhibition effect can be recovered by cytidine, for example, 5-fluorocytidine, 2-thiocytidine, 6-azacytidine, 3-deazauracil, 3-deazauridine and the like.

Examples of isolation of a DNA fragment containing CTP synthetase gene include a process wherein the chromosomal DNA is firstly extracted from the donor [for example, a method described in Biochim. Biophys. Acta, 72, 619 (1963) can be employed], and the resultant is cleaved with a suitable restriction enzyme. A typical example of DNA conferring cytidine analogue resistance includes DNA having two PstI cleavage sites at about 7.5 kb interval, and two EcoRI cleavage sites and one PvuII cleavage site in the PstI fragment.

Then, thus-obtained chromosomal DNA fragment containing CTP synthetase gene is inserted in a vector DNA.

The vector DNA used in the present invention is appropriately selected from those growable in a bacterial strain belonging to the genus Bacillus, or those growable in a bacterial strain belonging to the genus Escherichia and Bacillus (so-called shuttle vector) or a recombinant of vector DNA deficient in automonous replication ability in a host belonging to the genus Bacillus and chromosomal DNA of a bacterial strain belonging to the genus Bacillus by which the recombinant DNA can be stably inserted in chromosome of the host cell (so-called integration vector). The growable plasmids which can grow in a bacterial strain belonging to the genus Bacillus include, for example, pUB110 [J. Bacteriol., 134, 318 (1978)-], pC194 [Proc. Natl. Acad. Sci. U.S.A., 74, 1680 (1977)], pLS28 [J. Bacteriol., 131, 699 (1977)] and the like. The so-called shuttle vectors which can grow in a bacterial strain belonging to the both genera Escherichia and Bacillus include, for example, pHV14 [Proc. Natl. Acad. Sci. U.S.A., 75, 1433 (1978)], pHY300PLK [Jpn. J. Genet., 61, 515 (1986)] and the like. The integration vector includes pJH101 [J. Bacteriol., 154, 1513 (1983)], pGX345 [J. Bacteriol., 157, 718 (1984)] and the like. In addition to these known ones, the newly isolated or synthesized vector DNAs may be used so long as the objects of the present invention can be attained with them.

In order to insert DNA fragment containing CTP synthetase gene into these plasmid vectors, for examples the known method described in T. Maniatis et al., Molecular Cloning, A Laboratory Manual, Cold Spring Harbor Laboratory, Cold Spring Harbor, New York (1982) and the like can be employed.

Thus-obtained vector DNA integrating CTP synthetase gene can be used for transformation of a bacterial strain belonging to the genera Escherichia and Bacillus. For transformation of bacteria belonging to the genus Escherichia, a method wherein the recipient cells are treated with calcium chloride to introduce DNA [J. Mol. Biol., 53, 159 (1979)], that is, a competent cell method, can be used. For transformation of the bacteria belonging to the genus Bacillus, transformation methods, for example, a method wherein the recipient is converted to protoplast to introduce DNA [Mol. Gen. Genet., 168, 111 (1979)] or a method using competent cells [J. Bacteriol., 81, 714 (1961)] can be employed. The isolation of CTP synthetase gene includes, for example, a method wherein CTP synthetase defective mutant of bacteria belonging to the genus Bacillus or Escherichia is transformed and the strains which have CTP synthetase activity are isolated, and CTP synthetase gene is isolated from the resultant. This procedure based on the fact that CTP synthetase defective mutant can not grow on the medium containing no cytidine, while the transformant having vector DNA integrating CTP synthetase gene can grow on a medium containing no cytidine. The example of this recipient includes Escherichia coli G-1445. When the vector DNA to be examined has selected marker such as antibiotic tolerance, the method wherein the antibiotics may be added to the above selected medium or the like may be employed to further facilitate selection of the objective transformant. Thus-obtained vector DNA integrating CTP synthetase gene is extracted from a transformant and introduced into another recipient. Alternatively, a DNA fragment containing CTP synthetase gene is prepared from the extracted recombinant DNA, and the resultant is used combining with another vector plasmid. The host to which the plasmid is introduced may be a CTP synthetase defective strain, or a wild-type strain. In order to obtain a transformant having higher capability of the production of cytidine, a mutant or cytidine deaminase defective recombinant or transformant wherein the biosynthesis activity of cytidine or a precursor thereof is enhanced by imparting properties such as pyrimidine analogue resistance may be used as a recipient. The precursor used herein means carbamyl phosphate, carbamyl aspartate, aspartic acid, dihydroorotic acid, orotic acid, 5-phospho-D-ribosyldiphosphate, orotic acid, orotidine, orotidine 5'-monophosphate, uracil, uridine, uridine 5'-monophosphate, uridine 5'-diphosphate, uridine 5'-triphosphate and the like.

Examples of transformants thus obtained include, for example, Bacillus subtilis PINV128 (IFO 15028, FERM BP-2861) which is a transformant of Bacillus subtilis CDC-1, Bacillus subtilis PINV228 (IFO 15029, FERM BP-2859) which is a transformant of Bacillus subtilis DFCR-100 (IFO 15026, FERM BP-2860).

The aforementioned Escherichia coli G-1445 is derived from Escherichia coli C600 [Bacteriol. Rev., 36, 525 (1972)] as a parent strain, and which is deficient in CTP synthetase. Other mycological properties are the same as those of the parent strains. Bacillus subtilis DFCR-100 is derived from Bacillus subtilis No. 122 (IFO 14386, FERM P-7908) as a parent, deficient in cytidine deaminase, resistant to cytidine analogue and possesses capability of the production of cytidine. Other properties are the same as those of the parent strains. The IFO number used herein is the accession number in Institute for Fermentation (IFO, 2-17-85, Jusohonmachi, Yodogawa-ku, Osaka-city, Osaka, Japan) and FERM P number is the accession number in Fermentation Research Institute, Agency of Industrial Science and Technology (FRI, 1-1-3, Higashi, Tsukuba-city, Ibaragi, Japan) and FERM BP number is the accession number to FRI under Budapest treaty.

For cultivation of the above-obtained cytidine producing strain, there can be used cultivation methods for conventional microorganisms. That is, culture media containing carbon sources, nitrogen sources, metal ions and, if necessary, other nutrients such as amino acids, nucleic acids, vitamins and the like can be used. As the carbon sources, glucose, sucrose, maltose, sorbitol, starch, saccharified starch, molasses and the like can be used. As the nitrogen sources, in addition to organic nitrogen sources such as peptone, corn steep liquor, soybean meal, urea and the like, inorganic nitrogen sources such as ammonium salts of sulfuric acid, nitric acid, hydrochloric acid, carbonic acid, gaseous ammonia, aqueous ammonia and the like can be also used alone or in combination thereof. As other nutrients, various inorganic salts, amino acids, nucleic acid, vitamins and the like which are necessary for the growth of the microorganism are appropriately selected and used alone or in combination thereof. Further, if necessary, an antifoaming agent such as silicone oil, polyalkylene glycol ether or the like, a surfactant and the like can be added to the culture medium. Usually, the cultivation is carried out under aerobic conditions such as shaking culture, aeration submerged culture or the like. Preferably, pH of the culture medium is within the range of 4 to 9. When pH is varied during cultivation, sulfuric acid, calcium carbonate, sodium hydroxide, gaseous ammonia, aqueous ammonia and the like can be added to adjust pH to the above preferred range. Usually, the cultivation temperature is appropriately selected from the range of 20 to 45°C so that the temperature is suitable for the growth of a particular microorganism to be used and the accumulation of cytidine. The cultivation is continued until the accumulation of cytidine substantially becomes maximum. Usually, the object can be attained by cultivation for 2 to 6 days.

In order to isolate and obtain cytidine from a culture, there can be employed known purification means, for example, precipitation as well as isolation and purification methods such as chromatography using an ion exchange resin, activated charcoal and the like.

The following examples further illustrate the present invention in detail.

Example

(i) Derivation of cytidine producer

Bacillus subtilis No. 122 (IFO 14386, FERM P-7908) was treated with 50 μg/ml of N-methyl-N'-nitro-N-nitrosoguanidine (NTG) at 37°C for 20 minutes (hereinafter, NTG treatment was conducted under the same conditions), then spread on the following basal medium (MT-9) to which was added 0.01% uracil and cultivation was conducted at 37°C for 2 days. Uracil-requiring mutants were selected from the developed colony by replica method. Subsequently, the uracil-requiring mutant was subjected to NTG treatment and spread on MT-9 medium containing 0.01% uracil and cultivated at 37°C for 2 days.

## Basal medium (MT-9)

| | |
|---|---|
| Glucose | 0.5% |
| Potassium phosphate | 0.3% |
| Disodium phosphate | 0.6% |
| Ammonium chloride | 0.1% |
| Sodium chloride | 0.05% |
| Magnesium sulfate | 1.0mM |
| Calcium chloride | 0.1mM |
| Casamino acid | 0.3% |
| Agar | 2.0% |

(pH 7.2)

The developed colonies were replicated onto MT-9 medium to which 100 μg/ml cytidine was added to select cytidine deaminase defective strains which can not grow on this medium were selected. Subsequently, the above cytidine deaminase defective strains were subjected to NTG treatment, smeared on MT-9 medium and cultivated at 37°C for 2 days. Bacillus subtilis CDD-1 was selected as a cytidine deaminase defective strain from among the developed colonies. The CDD-1 was further treated with NTG, then smeared on MT-9 medium containing 5 μg/ml of 5-fluorocytidine which was a kind of cytidine analogues and cultivated at 37°C for 3 days. Bacillus subtilis FCR-13 was selected as a strain having capability of the production of cytidine from among the developed colonies. The FCR-13 was further treated with NTG, then smeared on MT-9 medium containing 1,000 μg/ml of 3-deazauracil which was a kind of cytidine analogues and cultivated 37°C for 3 days. Bacillus subtilis DFCR-100 (IFO 15026, FERM BP-2860) was selected as a strain having enhanced capability of the production of cytidine from among the developed colonies.

(ii) Preparation of chromosomal DNA

Bacillus subtilis DFCR-100 obtained in the above (i) having cytidine analogue tolerance and producing cytidine was inoculated on 1 liter of L medium shown below, cultivated at 37°C for 12 hours, and DNA was extracted from the resulting cells according to the method by Saito et al. [Biochim. Biophys. Acta, 72, 619 (1963)] to obtain 6.0 mg of chromosomal DNA.

## L medium

| | |
|---|---|
| Bactotrypton | 1.0% |
| Yeast Extract | 0.5% |
| Sodium Chloride | 0.5% |

(pH 7.0)

(iii) Insertion of chromosomal DNA into vector plasmid pBR322

5

The experiment described hereinafter was conducted according to the method described in T. Maniatis et al., Molecular Cloning, A Laboratory Manual, Cold spring Harbor Laboratory, Cold Spring Harbor, New York (1982), unless otherwise stated.

The chromosomal DNA obtained in the above (i) (10 μg) and pBR322 (3 μg) were respectively cleaved with the restriction enzyme PstI (manufactured by Nippon Gene Co., Ltd., Japan), then the both were mixed and ligated with DNA ligase (manufactured by Nippon Gene Co., Ltd.) derived from T4 phage.

(iv) Cloning of CTP synthetase gene providing cytidine analogue tolerance

According to the method employed for the production of the conventional auxotrophic mutant, cytidine-requiring strain, Escherichia coli G-1445 based on CTP synthetase deficiency was obtained by a combination of mutation with NTG and replica-plating method from Escherichia coli 600 [Bacteriol. Rev., 36, 525 (1972)].

For transformation of a recombinant plasmid, a method to introduce DNA by treatment of recipient cell with calcium chloride [J. Mol. Biol., 53, 159 (1979)], i.e., competent cell method was employed. The plasmid DNA prepared in the above (iii) was added to the suspension of competent cells prepared from Escherichia coli G-1445 for transformation. Subsequently, the suspension containing this transformant was smeared on MT-9 medium containing 12.5 μg/ml of tetracycline and cultivated at 37° C for 3 days. Escherichia coli G-1 (IFO 15027, FERM BP-2858) was obtained as a transformant having resistance to tetracycline and no requirement for cytidine was obtained from among the developed colonies.

(v) Extraction of plasmid from transformant

The transformant Escherichia coli G-1 was inoculated on 1 liter of L medium containing 12.5 μg/ml of tetracycline and cultivated at 37° C for 12 hours. Eventually, 500 μg of plasmid was isolated from the resulting strain and named as pYRG100.

(vi) Analysis of plasmid pYRG100

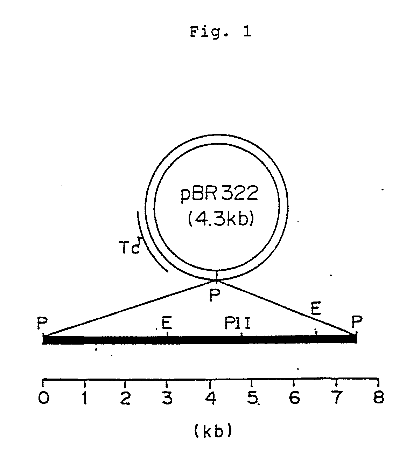

After cleaving pYRG100 with various restriction enzymes, agarose gel electrophoresis was carried out based on HindIII-digest of λ phage DNA (manufactured by Nippon Gene Co., Ltd., Japan) and restriction enzyme cleavage map shown in Fig. 1 was prepared from the resulting pattern. pYRG100 was a recombinant plasmid wherein about 7.5-kbp DNA fragment was inserted at PstI site of pBR322 and has EcoRI cleavage sites at 3.0, 6.5 kbp and PvuII cleavage site at 4.8 kbp from PstI site at the right side of the drawing.

(vii) Preparation of integration vector pINV100

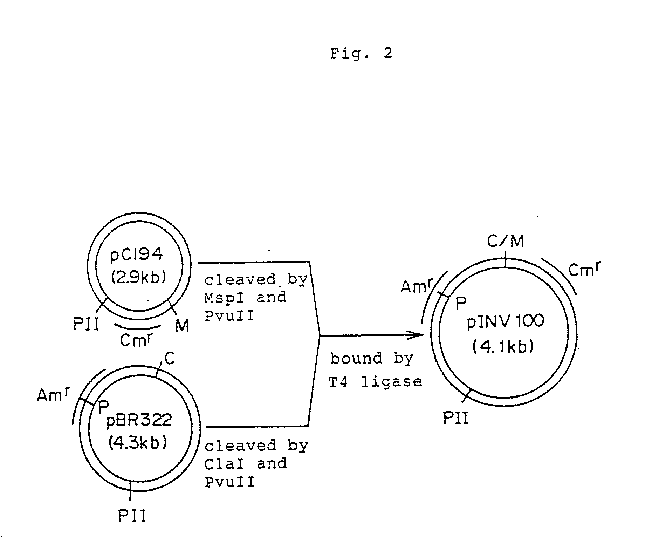

Chloramphenicol resistant gene carried by plasmid pC194 [J. Bacteriol., 150, 815 (1982)] was cleaved with restriction enzymes MspI and PvuII. After digests of plasmid pBR322 with ClaI and PvuII were mixed with the resultant, ligation was carried out using T4 ligase. The reaction solution was added to a suspension of competent cells prepared from Escherichia coli C600, and transformed according to the method shown in the above (iii). Then, the suspension containing this transformant was smeared on MT-9 medium containing 10 μg/ml of chloramphenicol and cultivated at 37° C for one day. The developed colonies were further replicated on MT-9 medium containing 50 μg/ml of ampicillin and cultivated at 37° C for one day. Thus, Escherichia coli PINV100 having resistance to chloramphenicol as well as resistance to ampicillin was obtained. A plasmid was extracted from Escherichia coli PINV100 by the method shown in the above (v) and named as pINV100. The preparation process and restriction enzyme map of pINV100 are shown in Fig. 2. In pINV100, chloramphenicol resistance gene derived from pC194 was inserted between ClaI and PvuII sites of pBR322.

(viii) Preparation of plasmid pINV128

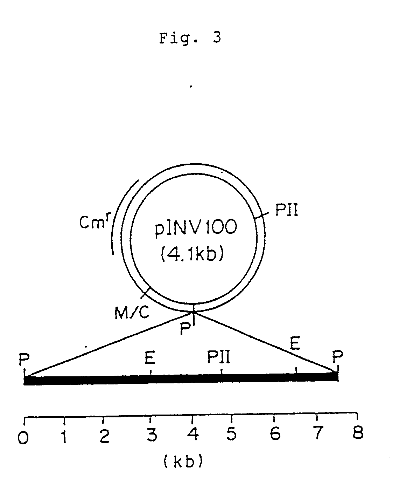

Plasmid pYRG100 (1 μg) obtained in the above (v) and plasmid pINV100 (1 μg) obtained in the above (vii) were cleaved with restriction enzyme PstI. After mixing, ligation was carried out using T4 ligase. Escherichia coli G-1445 was transformed in the same manner as that described in (iv) by using the resultant. Then, the suspension containing this transformant was smeared on MT-9 medium containing 10 μg/ml of chloramphenicol and cultivated at 37° C for 2 days. Escherichia coli PINV128 which was a transformant having chloramphenicol resistance and no requirement for cytidine was obtained in the developed colonies. A plasmid was extracted from Escherichia coli PINV128 in the same manner as that shown in the above (v), and the resultant was named as pINV128. Restriction enzyme map of pINV128 is shown in Fig. 3. In pINV128, CTP synthetase gene which provides cytidine analogue resistance is inserted at PstI site of pINV100.

(ix) Transformation of Bacillus subtilis with pINV128

Bacillus subtilis CDD-1 and Bacillus subtilis DFCR-100 were transformed using pINV128 obtained in (viii). Transformation was conducted according to a competent cell method [J. Bacteriol., 81, 714 (1961)], wherein MT-9 medium containing 10 μg/ml of chloramphenicol was used for selection of transformant. As transformants of Bacillus subtilis CDD-1 and Bacillus subtilis DFCR-100, Bacillus subtilis PINV128 (IFO 15028, FERM BP-2861) and Bacillus subtilis PINV228 (IFO 15029, FERM BP-2859) were obtained, respectively. There were no plasmids observed for Bacillus subtilis PINV128 and Bacillus subtilis PINV228. On the other hand, transformation using pINV100 without containing CTP synthetase gene, i.e., containing no DNA having homology to chromosomal DNA provided no chloramphenicol resistant strain. The results suggested that pINV128 was inserted in the host chromosome.

(x) Properties of the transformant with pINV128

Growth of the transformant PINV128 and its parent strain, CDD-1, on a medium containing chloramphenicol and cytidine analogue is shown in Table 1.

## Table 1

| Additives in MT-9 (μg/ml) | Growth of strain* CDD-1 | PINV128 |
|---|---|---|
| Nothing | + | + |
| Chloramphenicol (10) | − | + |
| 5-Fluorocytidine (5) | − | + |
| 3-Deazauracil (1000) | − | + |

*: +: growth observed, −: no growth observed

Strain PINV128 obtained by introducing plasmid pINV128 into CDD-1 shows resistance to cytidine analogue such as 5-fluorocytidine and 3-deazauracil. The result shows that the DNA fragment derived from pYRG100 which is inserted in pINV128 is CTP synthetase gene providing cytidine analogue resistance. Transformants PINV128 and PINV228 and their parent strains, i.e., CDD-1 and DFCR-100, were inoculated in a 200 ml volume flask containing the following production medium (20 ml), cultivated at 37° C for 3 days, and productivity of cytidine was compared. The results are shown in Table 2.

## Production Medium

| | |
|---|---|
| Glucose | 16.0% |
| Corn Steep Liquor | 4.0% |
| Urea | 2.0% |
| Calcium Carbonate | 0.5% |
| pH 7.0 | |

## Table 2

| Strain | Cytidine (mg/ml) |
|---|---|
| Bacillus subtilis CDD-1 | 0.1 |
| Bacillus subtilis PINV128 | 3.3 |
| Bacillus subtilis DFCR100 | 6.3 |
| Bacillus subtilis PINV228 | 8.2 |

As described hereinabove, according to the present invention, a cytidine producer can be derived by introducing the recombinant DNA containing CTP synthetase gene which provides cytidine analogue resistance.

Further, productivity of cytidine of a cytidine producer strain can be enhanced according to the present invention.

## Claims

1. DNA containing a gene region coding for cytidine 5'-triphosphate synthetase which is obtained from chromosomal DNA of bacterial strain of the genus Bacillus exhibiting cytidine analogue resistance.

2. DNA according to claim 1, wherein the cytidine 5'-triphosphate synthetase provides cytidine analogue resistance.

3. DNA according to claim 2 which has two PstI cleavage sites at about 7.5 kb interval, and two EcoRI cleavage sites and one PvuII cleavage site in the PstI fragment.

4. A plasmid in which the DNA according to any one of claims 1 to 3 is incorporated.

5. A bacterial strain transformed with the plasmid according to claim 4.

6. A bacterial strain according to claim 5 which belongs to the genus Bacillus and having capability of production of cytidine.

7. A process for production of cytidine which comprises cultivating a bacterial strain according to claim 6 in a culture medium to produce and accumulate cytidine in the culture medium, and collecting the resultant.

Fig. 1

Fig. 2

Fig. 3

European
Patent Office

# EUROPEAN SEARCH REPORT

Application Number

**EP 91 10 6397**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | JOURNAL OF BACTERIOLOGY vol. 170, no. 9, September 1988, pages 4194-4208, Baltimore, US; K. TRACH et al.: "Complete Sequence and Transcriptional Analysis of the spo0F Region of the Bacillus subtilis Chromosome" * whole article * | 1-5 | C 12 N 15/52 C 12 N 9/00 C 12 P 19/38 // (C 12 N 15/52 C 12 R 1:07 ) |
| Y | idem | 6,7 | |
| Y | EP-A-0 329 062 (TAKEDA CHEMICAL IND., LTD.) * whole document * | 6,7 | |
| A | EP-A-0 188 708 (TAKEDA CHEMICAL IND., LTD.) * whole document * | 1,7 | |
| A | FOLIA MICROBIOLOGICA vol. 17, no. 6, 1972, pages 517-521, Prague, CS; V. FUCIK et al.: "Mechanism of Resistance to 5-Azacytidine in Bacillus Subtilis. I. Isolation and some Properties of Mutants Resistant to 5-Azacytidine and 5-Aza-2'-deoxycytidine" * whole article * | 1-6 | |
| A | AGRICULTURAL AND BIOLOGICAL CHEMISTRY vol. 53, no. 1, 1989, pages 97-102, JP; S. ASAHI et al.: "Regulation of Pyrimidine Nucleotide Biosynthesis in Cytidine Deaminase-negative Mutants of Bacillus subtilis" * whole article * | 1 | |

**TECHNICAL FIELDS SEARCHED (Int. Cl.5)**

C 12 N 15/52
C 12 P 19/38
C 07 K 15/00

The present search report has been drawn up for all claims

| Place of search | Date of completion of search | Examiner |
|---|---|---|
| Berlin | 10 July 91 | JULIA P. |

CATEGORY OF CITED DOCUMENTS

X : particularly relevant if taken alone
Y : particularly relevant if combined with another
document of the same catagory
A : technological background
O : non-written disclosure
P : intermediate document
T : theory or principle underlying the invention

E : earlier patent document, but published on, or after
the filing date
D : document cited in the application
L : document cited for other reasons

&: member of the same patent family, corresponding
document